# EUROPÄISCHE PATENTSCHRIFT

(11) **EP 1 011 541 B1**
(45) Veröffentlichungstag und Bekanntmachung des Hinweises auf die Patenterteilung: **01.10.2003**
(21) Anmeldenummer: 95935792.2
(22) Anmeldetag: 09.11.1995
(51) Int. Cl.: A61F 2/36, A61F 2/46

(54) **FEMURKOMPONENTE FÜR EINE HÜFTENDOPROTHESE**
FEMUR COMPONENT FOR A HIP ENDOPROSTHESIS
COMPOSANT FEMORAL POUR ENDOPROTHESE DE LA HANCHE

(43) Veröffentlichungstag der Anmeldung: 28.06.2000
(73) Patentinhaber: Mathys Medizinaltechnik AG, 2544 Bettlach (CH)
(72) Erfinder: STUDER, Peter, CH-4552 Derendingen (CH); MATHYS, Robert, jun, CH-2544 Bettlach (CH); CLAES, Lutz, D-89233 Neu-Ulm (DE); KINZL, Lothar, D-89081 Ulm (DE); GAECHTER, André, CH-9402 Mörschwil (CH)
(74) Vertreter: Lusuardi, Werther Giovanni, Dr.
(86) Internationale Anmeldenummer: CH9500260
(87) Internationale Veröffentlichungsnummer: WO97017041

(56) Entgegenhaltungen:
- EP-A- 0 141 022
- EP-A- 0 273 871
- EP-A- 0 357 547
- EP-A- 0 423 064
- WO-A-95/11640
- DE-A- 3 829 361
- FR-A- 2 683 717
- US-A- 5 133 772

## Beschreibung

Die Erfindung bezieht sich auf eine Femurkomponente für eine Hüftendoprothese gemäss dem Oberbegriff des Patentanspruchs 1.

Eines der Probleme bei zementierten Femurkomponenten besteht darin, dass der im Markkanal eingeführte Knochenzement nach erfolgter Implantation des Prothesenschaftes nur mit zunehmender Kraftanwendung nach proximal verdrängt werden kann; dies gilt umsomehr wenn eine distale Markraumsperre verwendet wird.

Ein weiteres Problem besteht darin, dass die Femurkomponente bezogen auf den Prothesenschaft zu gross ausgeräumt werden muss, um für den Knochenzement Platz zu schaffen. In der sequenziellen Vorgehensweise wird der Schaft jeweils in das bereits mit Knochenzement gefüllte ausgeräumte Femurbett eingesetzt. Eine exakte und ruhige Lagerung während des Polymerisationsvorganges ist recht problematisch und kann bei unsachgemässer Handhabung zu ungleichförmiger Zementmantelbildung und lokal überhöhten Belastungskonzentrationen im ausgehärteten Knochenzement führen.

Hierzu ist eine gattungsgemässe Femurkomponente bereits aus der US 5,133,772 HACK ET AL. bekannt. Das Einfüllen des Knochenzementes wir bei dieser bekannten Femurkomponenten durch einen, bzw. mehrere interne Kanäle realisiert, welche vom Prothesenhals durch das Innere des Schaftes in eine peripher umlaufende Nut münden. Die Zementapplikation ist allerdings bei dieser bekannten Femurkomponente zu wenig gezielt lokal im proximalen Bereich, um einen möglichst physiologischen Lasttransfer zum Femur zu erzielen.
Die internen Kanäle führen zu einer mechanischen Schwächung des Implantates im Bereich des zu applizierenden Zementringes. weil die Kanülierung den Querschnitt des Prothesenschaftes schwächt. Die lange und gewundene Kanülierung in der Prothese beschränkt die Verwendung von normal viskosem Knochenzement auf eine zeitlich sehr kurze Phase der Polymerisation. Wird diese Phase überschritten, besteht die Gefahr eines übergrossen Reibwiderstandes des zu applizierenden Knochenzementes in den Kanälen; dies kann zu einer qualitativen Beeinträchtigung des auspolymerisierten proximalen Zementringes führen. Schliesslich bergen die internen Kanäle auch die Gefahr einer Verstopfung in sich.

Die standardmässig zementierten Systeme führen insofern zu einer wenig gezielten Krafteinleitung auf das Femur als die Lasteinleitung über den ganzen, aber vorwiegend distalen Prothesenschaft erfolgt und damit unphysiologisch ist. Dies kann zu einer proximalen Knochenresorption und späteren Instabilität der Prothese führen.

Hier will die Erfindung Abhilfe schaffen. Der Erfindung liegt die Aufgabe zugrunde eine Femurkomponente für eine Hüftendoprothese zu schaffen, welche über eine kompakte homogene Zementmantelzone proximal im Femur fixierbar ist, dabei eine weitgehend physiologische Lastverteilung im proximalen Schaftbereich erzielt und die Gefahr einer varusmässige Abkippung weitgehend eliminiert.

Die Erfindung löst die gestellte Aufgabe mit einer Femurkomponente, welche die Merkmale des Anspruchs 1 aufweist.
Die Erfindung kombiniert die Vorteile einer Zementverankerung, wie bessere Primärstabilität durch den initialen guten Formschluss, mit der proximalen Verankerung und damit physiologischen Belastung des Femurs.
Mittels einer angepassten Operationstechnik, beziehungsweise Implantatbettaufbereitung kann mit der erfindungsgemässen Femurkomponente die Passgenauigkeit nicht zementierter Systeme erreicht werden, was eine minimale Ausräumung des Femurs gestattet.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Femurkomponente eine lokale Zementapplikation im proximalen Bereiche des Femurs während eines erheblichen Polymerisationszeitraumes möglich ist.

Die Erfindung und Weiterbildungen der Erfindung werden im folgenden anhand der teilweise schematischen Darstellungen mehrerer Ausführungsbeispiele noch näher erläutert.
Es zeigen:
Fig. 1 eine perspektivische Darstellung der erfindungsgemässen Femurkomponente; und
Fig. 2 eine Seitenansicht der Femurkomponente nach Fig. 1.

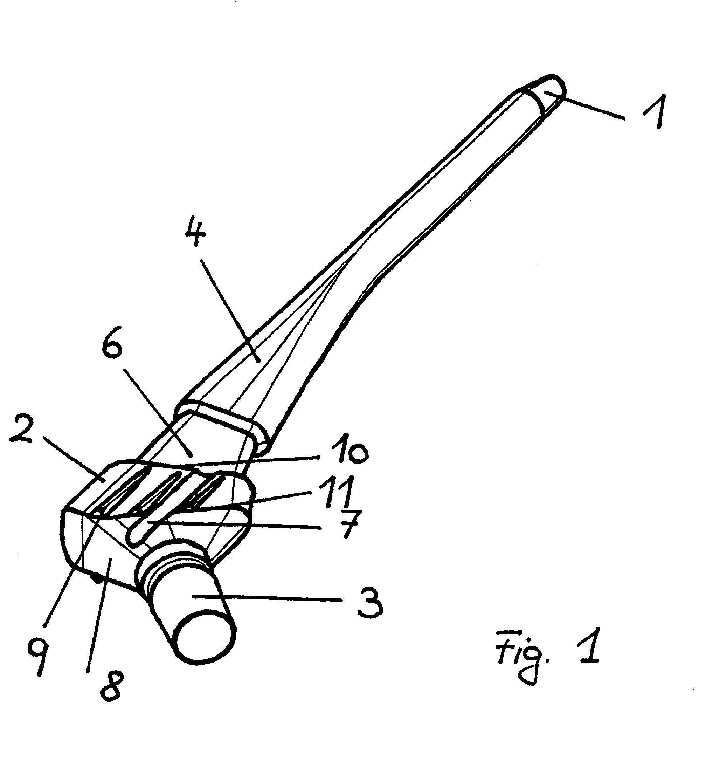

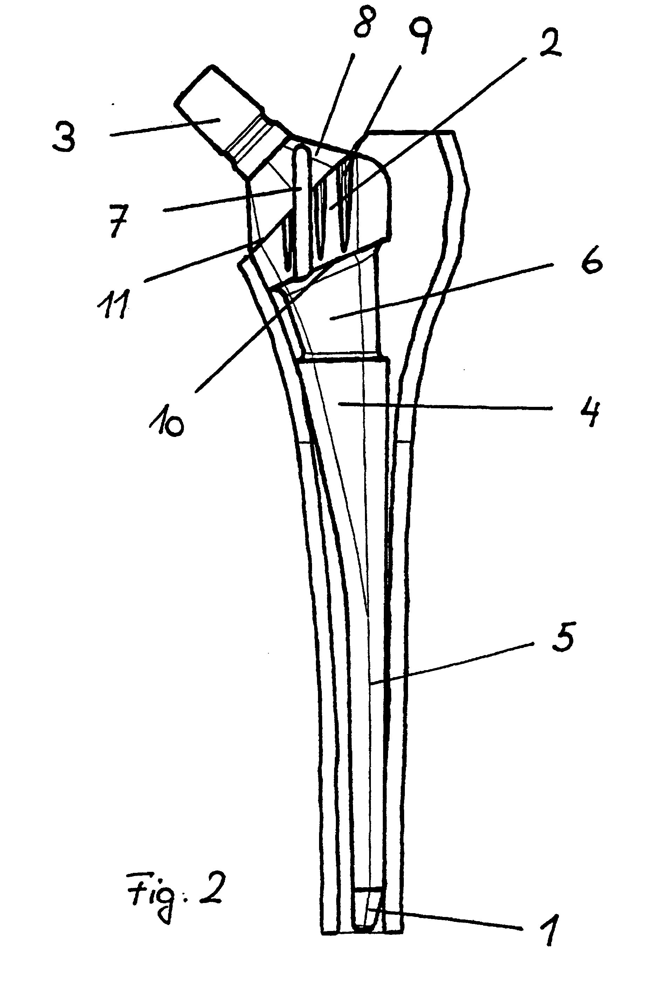

Die in den Fig. 1 und 2 dargestellt Femurkomponente für eine Hüftendoprothese weist ein zur Einführung in den Markraum des Femur bestimmtes distales Ende 1, eine proximale Schulterpartie 2 mit Hals 3, eine zwischen Schulterpartie 2 und Ende 1 liegenden Schaftteil 4 mit Längsachse 5 und Länge L, sowie eine, im proximalen, in die Schulterpartie 2 übergehenden Drittel des Schaftteils 4 angebrachte, peripher umlaufende Nut 6 auf.

Die Nut 6 ist über zwei oder mehrere in Richtung der Längsachse 5 auf der Oberfläche der Schulterpartie 2 verlaufende Kanäle 7 mit der proximalen Zone 8 der Schulterpartie 2 verbunden. In Fig. 2 ist ein anterior liegender Kanal 7 sichtbar. Ein entsprechender Kanal ist auch posterior angebracht. Die Kanäle 7 weisen eine mittlere Querschnittsfläche von 8 bis 120 mm², vorzugsweise von 15 bis 50 mm² auf.

Die Nut 6 weist zweckmässigerweise eine mittlere Tiefe von 0,5 bis 6,0 mm, vorzugsweise von 1 bis 4 mm auf, beziehungsweise von 2 - 40 % der in medio-lateraler Richtung gemessenen Breite der Schulterpartie 2.
Die mittlere Höhe der Nut 6 beträgt, in Richtung der Längsachse 5 gemessen, zweckmässigerweise 10 bis 50 mm, vorzugsweise 15 bis 35 mm, beziehungsweise 5 - 25 % der Länge L.
Das Volumen der Nut 6 beträgt zweckmässigerweise 500 bis 10'000 mm³, vorzugsweise 2'000 bis 6'000 mm³.

Der mittlere Querschnitt des Schaftteils 4 im Bereich der Nut 6 beträgt vorteilhafterweise 50 bis 500 mm², vorzugsweise 100 bis 300 mm².
Das Zentrum der Nut 6 weist einen Abstand von 60 bis 250 mm, vorzugsweise 100 bis 200 mm zum distalen Schaftende 1 auf und von 15 bis 100 mm, vorzugsweise von 30 bis 60 mm zum distalen Ende des Halses 3 auf.
Die proximale Grenze 10 der Nut 6 weist medial gemessen einen Abstand von 2 bis 18 mm, vorzugsweise von 5 bis 10 mm zur distalen Grenze 11 der proximalen Zone 8 der Schulterpartie 2 auf. Lateral gemessen weist die proximale Grenze 10 der Nut 6 einen Abstand von 5 bis 50 mm, vorzugsweise von 15 bis 35 mm zur distalen Grenze 11 der proximalen Zone 8 der Schulterpartie 2 aufweist.

Der mittlere Querschnitt des Schaftteils 4 beträgt in seinem distalen, das Ende 1 einschliessende Drittel vorteilhafterweise 25 bis 120 mm², vorzugsweise 40 bis 80 mm².

Im proximalen, in die Schulterpartie 2 übergehenden Drittel des Schaftteils 4 können - zusätzlich zu den Kanälen 7 - nicht durchgehende Rillen oder Rippen 9 angebracht sein. Der Vorteil dieser zusätzlichen Rillen oder Rippen 9 liegt im damit erzielbaren formschlüssigen Verbund zwischen Prothese und Femur zur Verbesserung der Primärstabilität im Sinne einer nichtzementierten Prothese. Dies führt zu einer weiteren (zusätzlich zur Zementverankerung wirkenden) makroskopischen spongiösen Verankerung, welche die drehmomentmässige Belastbarkeit (Rotationsstabilität, Rotationssicherung) der Prothese erhöht.
Die zusätzlichen Rillen oder Rippen 9 ergeben zudem eine zusätzliche Führung und somit eine exakte lagemässige Positionierung der Prothese während des Setzvorganges.

In der proximalen Hälfte der Femurkomponente, beinhaltend die Schulterpartie 2 und den proximalen Teil des Schaftteils 4, aber ohne Nut 6, ist eine Mikrostrukturierung vorgesehen, welche eine mittlere Rauhtiefe von 5 µm bis 1000 µm, vorzugsweise von 10 µm bis 600 µm aufweist. Da die gesamte proximale Partie zur Krafteinleitung vom Implantat zum Knochen genutzt wird und eine proximale Zone oberhalb des Zementringes für Abstütz- und Führungsaufgaben vorgesehen ist, erfolgt insbesondere in dieser Zone mittels einer entsprechenden Oberflächenbeschaffenheit eine Optimierung des knöchernen Anwachsverhaltens und damit eine knöcherne Integration. Vorteilhafterweise ist diese proximale Hälfte der Femurkomponente (ohne Nut 6) mindestens teilweise mit einer Beschichtung aus einem bioaktivem Material, vorzugsweise Hydroxylapatit versehen. Die damit erzielbare mikroskopische Verankerung - als Überlagerung zur makroskopischen Verankerung - verbessert die Implantathaftung und knöcherne Integration und damit die Langzeitstabilität. Wenn die Beschichtung aus bioaktivem Material ist, führt dies zu einer Verbesserung der Primärstabilität.

Am Hals 3 ist in bekannter Weise ein Kugelkopf befestigbar.

## Patentansprüche

1. Femurkomponente für eine Hüftendoprothese mit einem distalen zur Einführung in den Markraum des Femur bestimmten Ende (1), einer proximalen Schulterpartie (2) mit einer proximalen zone (8) und mit einem Hals (3), an welchen ein Kugelkopf befestigbar ist, einem zwischen Schulterpartie (2) und Ende (1) liegenden Schaftteil (4) mit Längsachse (5) und Länge L, sowie einer, im proximalen, in die Schulterpartie (2) übergehenden Drittel des Schaftteils (4) angebrachten, peripher umlaufenden Nut (6),
**dadurch gekennzeichnet, dass**
die Nut (6) über mindestens zwei auf der Oberfläche der Schulterpartie (2) verlaufende Kanäle (7) mit der proximalen Zone (8) der Schulterpartie (2) verbunden ist.

2. Femurkomponente nach Anspruch 1, **dadurch gekennzeichnet, dass** im proximalen, in die Schulterpartie (2) übergehenden Drittel des Schaftteils (4), zusätzlich zu den Kanälen (7), nicht durchgehende Rillen oder Rippen (9) vorgesehen sind.

3. Femurkomponente nach Anspruch 1 oder 2, **dadurch gekennzeichnet, dass** die proximale Hälfte der Femurkomponente, vorzugsweise die darin enthaltene Schulterpartie (2) eine Mikrostrukturierung aufweist.

4. Femurkomponente nach einem der Ansprüche 1 bis 3, **dadurch gekennzeichnet, dass** die proximale Hälfte der Femurkomponente, vorzugsweise die darin enthaltene Schulterpartie (2) eine mindestens teilweise Beschichtung aus einem bioaktivem Material, vorzugsweise Hydroxylapatit aufweist.

5. Femurkomponente nach einem der Ansprüche 1 - 4, **dadurch gekennzeichnet, dass** die Nut (6) eine mittlere Tiefe von 0,5 bis 6,0 mm, vorzugsweise von 1 bis 4 mm aufweist.

6. Femurkomponente nach einem der Ansprüche 1 - 4, **dadurch gekennzeichnet, dass** die Nut (6) eine mittlere Tiefe von 2 - 40 % der in medio-lateraler Richtung gemessenen Breite der Schulterpartie (2) aufweist.

7. Femurkomponente nach einem der Ansprüche 1 - 6, **dadurch gekennzeichnet, dass** die Nut (6) eine in Richtung der Längsachse (5) gemessene mittlere Höhe von 10 bis 50 mm, vorzugsweise von 15 bis 35 mm aufweist.

8. Femurkomponente nach einem der Ansprüche 1 - 6, **dadurch gekennzeichnet, dass** die Nut (6) eine in Richtung der Längsachse (5) gemessene mittlere Höhe von 5 - 25 % der Länge L aufweist.

9. Femurkomponente nach einem der Ansprüche 1 bis 8, **dadurch gekennzeichnet, dass** das Volumen der Nut (6) 500 bis 10'000 mm³, vorzugsweise 2'000 bis 6'000 mm³ beträgt.

10. Femurkomponente nach einem der Ansprüche 1 -bis 9, **dadurch gekennzeichnet, dass** der mittlere Querschnitt des Schaftteils (4) im Bereich der Nut (6) 50 bis 500 mm², vorzugsweise 100 bis 300 mm² beträgt.

11. Femurkomponente nach einem der Ansprüche 1 bis 10, **dadurch gekennzeichnet, dass** das Zentrum der Nut (6) einen Abstand von 60 bis 250 mm, vorzugsweise 100 bis 200 mm zum distalen Schaftende (1) aufweist.

12. Femurkomponente nach einem der Ansprüche 1 bis 10, **dadurch gekennzeichnet, dass** das Zentrum der Nut (6) einen Abstand von 15 bis 100 mm, vorzugsweise von 30 bis 60 mm zum distalen Ende des Halses (3) aufweist.

13. Femurkomponente nach einem der Ansprüche 1 bis 12, **dadurch gekennzeichnet, dass** der mittlere Querschnitt des Schaftteils (4) in seinem distalen, das Ende (1) einschliessende Drittel 25 bis 120 mm², vorzugsweise 40 bis 80 mm² beträgt.

14. Femurkomponente nach einem der Ansprüche 3 - 13, **dadurch gekennzeichnet, dass** die Mikrostrukturierung eine mittlere Rauhtiefe von 5 µm bis 1000 µm, vorzugsweise von 10 µm bis 600 µm aufweist.

15. Femurkomponente nach einem der Ansprüche 1 bis 14, **dadurch gekennzeichnet, dass** die Kanäle (7) eine mittlere Querschnittsfläche von 8 bis 120 mm², vorzugsweise von 15 bis 50 mm² aufweisen.

16. Femurkomponente nach einem der Ansprüche 1 bis 15, **dadurch gekennzeichnet, dass** die proximale Grenze (10) der Nut (6) medial gemessen einen Abstand von 2 bis 18 mm, vorzugsweise von 5 bis 10 mm zur distalen Grenze (11) der proximalen Zone (8) der Schulterpartie (2) aufweist.

17. Femurkomponente nach einem der Ansprüche 1 bis 16, **dadurch gekennzeichnet, dass** die proximale Grenze (10) der Nut (6) lateral gemessen einen Abstand von 5 bis 50 mm, vorzugsweise von 15 bis 35 mm zur distalen Grenze (11) der proximalen Zone (8) der Schulterpartie (2) aufweist.

## Claims

1. Femur component for a hip endoprosthesis with a distal end (1 ) destined for insertion into the femur medullary space, a proximal shoulder section (2) with a proximal zone (8) and with a neck (3) whereon a spherical head is attachable, a shaft section (4) with a longitudinal axis (5) and a length L lying between the shoulder section (2) and the end (1), and a circumferential groove (6) in the proximal third of the shaft section (4) merging into the shoulder section (2),
**characterised in that**
the groove (6) is linked to the proximal zone (8) of the shoulder section (2) via at least two channels (7) on the surface of the shoulder section (2).

2. Femur component according to claim 1, **characterised in that** additionally to the channels (7) grooves or fins (9) which are not passing through are provided in the proximal third of the shaft section (4) which merges into the shoulder section (2).

3. Femur component according to claim 1 or 2, **characterised in that** the proximal half of the femur component preferably the shoulder section (2) included provides a microstructure.

4. Femur component according to one of the claims 1 to 3, **characterised in that** the proximal half of the femur component preferably the shoulder section (2) included provides at least partially a coating with a bioactive material preferably with hydroxylapatite.

5. Femur component according to one of the claims 1 to 4, **characterised in that** the groove (6) provides an average depth amounting between 0,5 and 6,0 mm preferably between 1 to 4 mm.

6. Femur component according to one of the claims 1 to 4, **characterised in that** the groove (6 ) provides an average depth with an amount of 2 - 40% of the width of the shoulder section (2) measured in medio-lateral direction.

7. Femur component according to one of the claims 1 to 6, **characterised in that** the groove (6) provides an average height in the direction of the longitudinal axis (5) amounting between 10 to 50 mm preferably 15 to 35 mm.

8. Femur component according to one of the claims 1 to 6, **characterised in that** the groove (6) provides an average height in the direction of the longitudinal axis (5) with an amount of 5 - 25% of the length L.

9. Femur component according to one of the claims 1 to 8, **characterised in that** the volume of the groove (6) amounts to 500 to 10'000 mm³, preferably 2'000 to 6'000 mm³.

10. Femur component according to one of the claims 1 to 9, **characterised in that** the average cross-section area of the shaft section (4) in the range of the groove (6) amounts to 50 to 500 mm² preferably to 100 to 300 mm².

11. Femur component according to one of the claims 1 to 10, **characterised in that** the center of the groove (6) provides a distance to the distal shaft end (1 ) amounting to 60 to 250 mm preferably 100 to 200 mm.

12. Femur component according to one of the claims 1 to 10, **characterised in that** the center of the groove (6) provides a distance to the distal end of the neck (3) amounting to 15 to 100 mm preferably 30 to 60 mm.

13. Femur component according to one of the claims 1 to 12, **characterised in that** the average cross-section area of the shaft section (4) in its distal end third which includes the end (1) amounts to 25 to 120 mm² preferably 40 to 80 mm².

14. Femur component according to one of the claims 3 to 13, **characterised in that** the microstructure provides an average surface roughness amounting to 5 µm to 1000 µm preferably to 10 µm to 600 µm.

15. Femur component according to one of the claims 1 to 14, **characterised in that** the channels (7) provide an average cross-section area that amounts to 8 to 120 mm² preferably to 15 to 50 mm².

16. Femur component according to one of the claims 1 to 15, **characterised in that** the proximal boundary (10) of the groove (6) provides a medially measured distance to the distal boundary (11) of the proximal zone (8) of the shoulder section (2) that amounts to 2 to 18 mm preferably to 5 to 10 mm.

17. Femur component according to one of the claims 1 to 16, **characterised in that** the proximal boundary (10) of the groove (6) provides a laterally measured distance to the distal boundary (11) of the proximal zone (8) of the shoulder section (2) which amounts to 5 to 50 mm preferably to 15 to 35 mm.

## Revendications

1. Composant fémoral pour une endoprothèse de la hanche, qui présente une extrémité distale (1) destiné à être insérée dans l'espace médullaire du fémur, une partie proximale d'épaulement (2) dotée d'une zone proximale (8) et d'un col (3) sur lequel une tête sphérique peut être fixée, une partie de bras (4) située entre la partie d'épaulement (2) et l'extrémité (1), qui présente un axe longitudinal (5) et une longueur L ainsi qu'une rainure périphérique continue (6) située dans le tiers proximal de la partie de bras (4) qui se prolonge en la partie d'épaulement (2), **caractérisé en ce que** la rainure (6) est reliée à la zone proximale (8) de la partie d'épaulement (2) par au moins deux canaux (7) qui s'étendent à la surface de la partie d'épaulement (2).

2. Composant fémoral selon la revendication 1, **caractérisé en ce qu'**en plus des canaux (7), des rainures ou nervures (9) non continues sont prévues dans le tiers de la partie de bras (4) qui se prolonge en la partie d'épaulement (2).

3. Composant fémoral selon la revendication 1 ou 2, **caractérisé en ce que** la moitié proximale du composant de fémur, de préférence la partie d'épaulement (2) qui y est contenue, présente une microstructuration.

4. Composant fémoral selon l'une des revendications 1 à 3, **caractérisé en ce que** la moitié proximale du composant fémoral, de préférence la partie d'épaulement (2) qui y est contenue, présente au moins en partie un revêtement constitué d'un matériau bioactif, de préférence de l'hydroxyapatite.

5. Composant fémoral selon l'une des revendications 1 à 4, **caractérisé en ce que** la rainure (6) présente une profondeur moyenne de 0,5 à 6,0 mm et de préférence de 1 à 4 mm.

6. Composant fémoral selon l'une des revendications 1 à 4, **caractérisé en ce que** la rainure (6) présente une profondeur moyenne qui représente de 2 à 40 % de la largeur de la partie d'épaulement (2) mesurée dans la direction médio-latérale.

7. Composant fémoral selon l'une des revendications 1 à 6, **caractérisé en ce que** la rainure (6) présente une hauteur moyenne mesurée dans la direction de l'axe longitudinal (5) de 10 à 50 mm et de préférence de 15 à 35 mm.

8. Composant fémoral selon l'une des revendications 1 à 6, **caractérisé en ce que** la rainure (6) présente une hauteur moyenne mesurée dans la direction de l'axe longitudinal (5) qui représente de 5 à 25 % de la longueur L.

9. Composant de fémur selon l'une des revendications 1 à 8, **caractérisé en ce que** le volume de la rainure (6) est de 500 à 10 000 mm³ et de préférence de 2 000 à 6 000 mm³.

10. Composant fémoral selon l'une des revendications 1 à 9, **caractérisé en ce que** la section transversale moyenne de la partie de bras (4) dans la région de la rainure (6) est de 50 à 500 mm² et de préférence de 100 à 300 mm².

11. Composant fémoral selon l'une des revendications 1 à 10, **caractérisé en ce que** le centre de la rainure (6) présente par rapport à l'extrémité distale (1) du bras une distance de 60 à 250 mm et de préférence de 100 à 200 mm.

12. Composant fémoral selon l'une des revendications 1 à 10, **caractérisé en ce que** le centre de la rainure (6) présente par rapport à l'extrémité distale du col (3) une distance de 15 à 100 mm et de préférence de 30 à 60 mm.

13. Composant fémoral selon l'une des revendications 1 à 12, **caractérisé en ce que** la section transversale moyenne de la partie de bras (4) dans son tiers distal qui comprend l'extrémité (1) est de 25 à 120 mm² et de préférence de 40 à 80 mm².

14. Composant fémoral selon l'une des revendications 3 à 13, **caractérisé en ce que** la microstructuration présente une rugosité d'une profondeur moyenne de 5 um à 1 000 µm et de préférence de 10 µm à 600 um.

15. Composant fémoral selon l'une des revendications 1 à 14, **caractérisé en ce que** les canaux (7) présentent une section transversale d'une surface moyenne de 8 à 120 mm² et de préférence de 15 à 50 mm².

16. Composant fémoral selon l'une des revendications 1 à 15, **caractérisé en ce que** la limite proximale (10) de la rainure (6) mesurée dans le sens médial présente par rapport à la limite distale (11) de la zone proximale (8) de la partie d'épaulement (2) une distance de 2 à 18 mm et de préférence de 5 à 10 mm.

17. Composant fémoral selon l'une des revendications 1 à 16, **caractérisé en ce que** la limite proximale (10) de la rainure (6) mesurée dans le sens latéral présente par rapport à la limite distale (11) de la zone proximale (8) de la partie d'épaulement (2) une distance de 5 à 50 mm et de préférence de 15 à 35 mm.
